(19) Europäisches Patentamt
European Patent Office
Office européen des brevets

(11) Veröffentlichungsnummer: **0 209 700**
**B1**

(12) **EUROPÄISCHE PATENTSCHRIFT**

(45) Veröffentlichungstag der Patentschrift: **01.08.90**

(21) Anmeldenummer: **86107841.8**

(22) Anmeldetag: **09.06.86**

(51) Int. Cl.$^5$: **C 07 C 275/18,**
C 07 C 273/18, C 07 C 271/10,
C 07 C 269/02, A 61 K 6/02,
C 08 F 20/36, C 08 F 22/22

# (54) (Meth)-acrylsäure-Derivate von Tricyclodecanen und ihre Verwendung.

(30) Priorität: **20.06.85 DE 3522006**

(43) Veröffentlichungstag der Anmeldung:
**28.01.87 Patentblatt 87/05**

(45) Bekanntmachung des Hinweises auf die Patenterteilung:
**01.08.90 Patentblatt 90/31**

(84) Benannte Vertragsstaaten:
**AT BE CH DE FR GB IT LI NL SE**

(56) Entgegenhaltungen:
**EP-A-0 023 685**
**EP-A-0 023 686**
**EP-A-0 171 847**
**EP-A-0 206 074**
**DE-A-2 816 823**

(73) Patentinhaber: **BAYER AG**
**D-5090 Leverkusen 1 Bayerwerk (DE)**

(72) Erfinder: **Winkel, Jens, Dr.**
**Hahnenweg 6**
**D-5000 Köln 80 (DE)**
Erfinder: **Bömer, Bruno, Dr.**
**Max-Planck-Strasse 53**
**D-5060 Bergisch Gladbach 2 (DE)**
Erfinder: **Schmitz-Josten, Robert, Dr.**
**Gerstenkamp 6**
**D-5000 Köln 80 (DE)**
Erfinder: **Klein, Gerhard, Dr.**
**von Flotow-Strasse 7**
**D-4019 Monheim (DE)**
Erfinder: **Süling, Carlhans, Dr.**
**Carl-Leverkus-Strasse 10**
**D-5068 Odenthal (DE)**
Erfinder: **Arlt, Dieter, Prof. Dr.**
**Rybniker Strasse 2**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 209 700 B1

**Beschreibung**

Die Erfindung betrifft neue (Meth)-acrylsäurederivate von Tricyclodecanen, ihre Herstellung und ihre Verwendung als Monomere für Dentalmaterialien.

Aus der DE—OS 2 931 926 sind Dentalmassen bekannt, die polymerisierbare (Meth)-acrylsäureester von Tricyclodecanan enthalten. Diese Dentalmassen zeigen bei der Anwendung jedoch immer noch einen Polymerisationsschrumpf.

Es wurden neue (Meth)-acrylsäure-Derivate von Tricyclodecanen der Formel

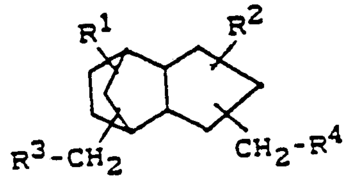

(I)

in der

$R^1$ und $R^2$ gleich oder verschieden sind und Wasserstoff, Niederalkyl Niederalkoxy, Halogen oder Trifluormethyl bedeuten, und

$R^3$ und $R^4$ gleich oder verschieden sind und für die Gruppe

$$-Y-O-\underset{\underset{O}{\|}}{C}-\underset{\underset{R^5}{|}}{C}=CH_2$$

stehen, wobei

X ein zweibindiges Brückenglied aus der Gruppe

$-O-\underset{\underset{O}{\|}}{C}-NH-$ oder $-\underset{\underset{R^6}{|}}{N}-\overset{\overset{O}{\|}}{C}-NH-$ und

Y ein zweibindiges Brückenglied aus der Gruppe

$-CH_2-CH_2-$ oder $-\underset{\underset{CH_3}{|}}{CH}-\overset{\overset{CH_3}{|}}{CH}-CH_2-$ oder 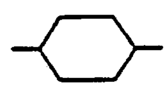

bedeutet,

und wobei

$R^5$ für Wasserstoff oder Methyl und

$R^6$ für Wasserstoff, Niederalkyl oder Phenyl stehen, gefunden,

Eine weitere Gruppe derartiger (Meth)-acrylsäure-Derivate wird in der parallelen EP—A—0 206 074 beschrieben.

Dentalmassen, bei denen von den erfindungsgemäßen (Meth)-acrylsäure-Derivaten von Tricyclodecanen ausgegangen wird, zeigen überraschenderweise einen wesentlich geringeren Polymerisationsschrumpf und sind daher für die Anwendung in der Praxis besonders geeignet.

Im Rahmen der vorliegenden Erfindung können die Substituenten die folgende Bedeutung haben:

Niederalkyl kann einen geradkettigen oder verzweigten Kohlenwasserstoffrest mit 1 bis etwa 6 Kohlenstoffatomen bedeuten. Beispielsweise seien die folgenden Niederalkylreste genannt: Methyl, Ethyl, Propyl, Isopropyl, Butyl, Isobutyl, Pentyl, Isopentyl, Hexyl und Isohexyl. Bevorzugt werden der Methyl- und der Ethylrest.

Niederalkoxy kann einen über Sauerstoff gebundenen geradkettigen oder verzweigten Kohlenwasserstoffrest mit 1 bis etwa 6 Kohlenstoffatomen bedeuten. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugt werden der Methoxy- und der Ethoxyrest.

Halogen kann Fluor, Chlor, Brom und Jod bedeuten. Bevorzugt werden Fluor und Chlor.

Bevorzugt werden (Meth)-acrylsäure-Derivate von Tricyclodecane der Formel 1, bei denen

$R^1$ und $R^2$ Wasserstoff bedeuten,

$R^3$ und $R^4$ gleich oder verschieden sind und für die Gruppe

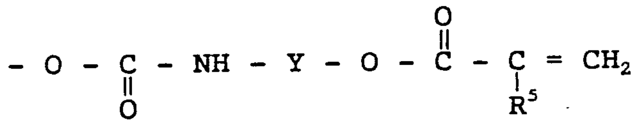

stehen, wobei

Y ein zweibindiges Brückenglied aus der Gruppe

$-CH_2-CH_2-$ oder 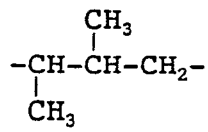 oder 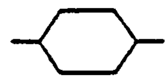

bedeutet,

und wobei

$R^5$ für Wasserstoff oder Methyl steht.

Beispielsweise seien die folgenden (Meth)-acrylsäure-Derivate von Tricyclodecanen genannt:

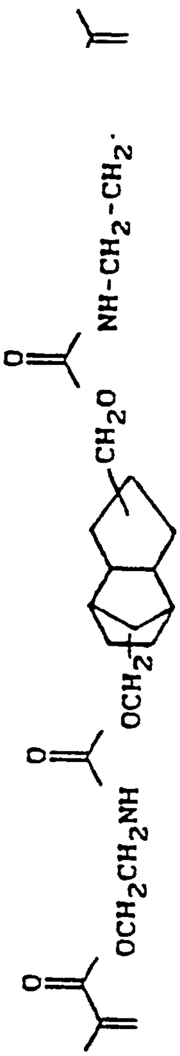

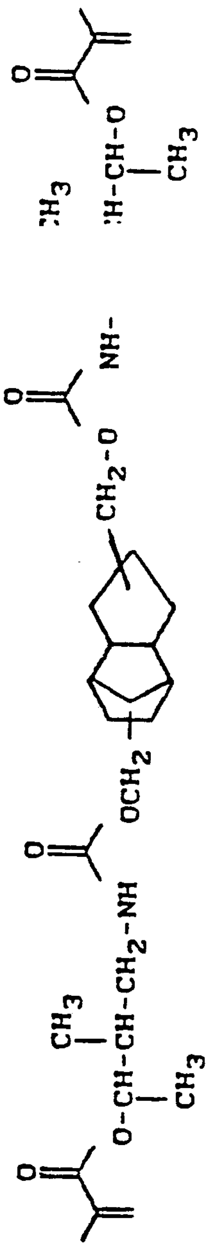

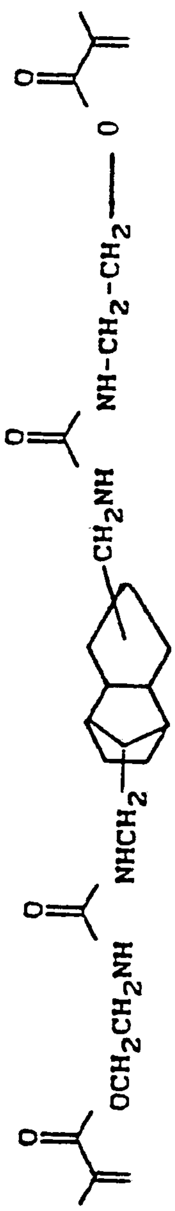

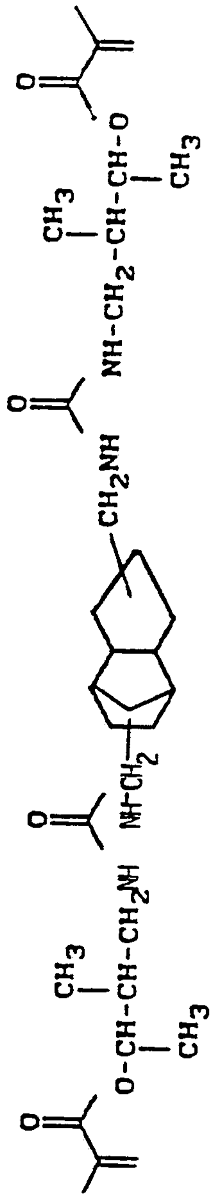

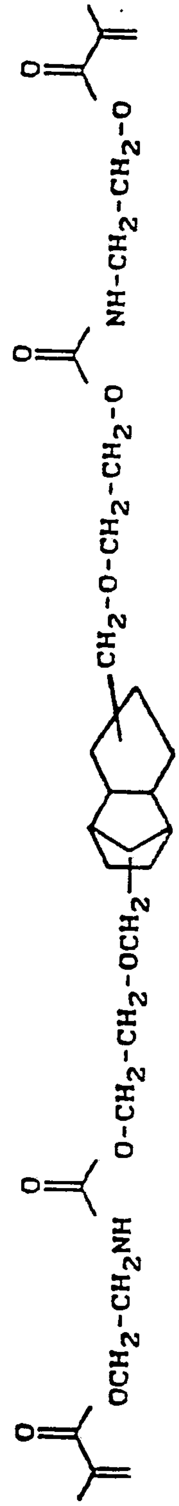

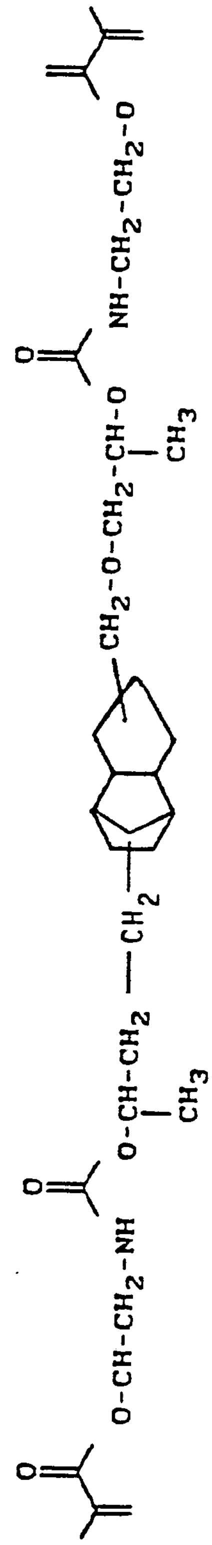

Es wurde auch ein Verfahren zur Herstellung (Meth)-acrylsäure-Derivaten von Tricyclodecan gefunden, das dadurch gekennzeichnet ist, daß man Tricyclodecane der Formel

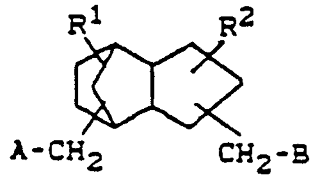

(II)

in der

$R^1$ und $R^2$ gleich oder verschieden sind und Wasserstoff, Niederalkyl Niederalkoxy, Halogen oder Trifluormethyl bedeuten, und

A und B gleich oder verschieden sind und Hydroxy oder den Rest —$NHR^6$ bedeuten, wobei $R^6$ für Wasserstoff, Niederalkyl oder Phenyl steht,

mit (Meth)-acrylsäureester-Isocyanaten der Formel

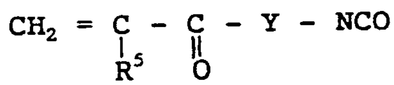

(III),

$-CH_2-CH_2-$ oder 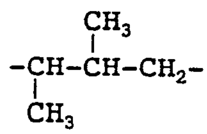 oder 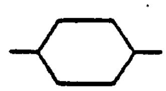

bedeutet,

gegebenenfalls in einem inerten Lösungsmittel in Gegenwart eines Katalysators im Temperaturbereich von 0 bis 100°C umsetzt.

Die als Ausgangsverbindungen eingesetzten Dihydroxymethyltricyclodecane sind an sich bekannt (Literatur USP 4,131,729) und können beispielsweise durch Umsetzung von Dicyclopentadien, Formaldehyd und Wasserstoff hergestellt werden.

Die als Ausgangsverbindungen eingesetzten Diaminomethyltricyclodecane sind an sich bekannt und können beispielsweise durch Umsetzung von Tosylaten mit Ammoniak hergestellt werden.

(Meth)-acrylsäureester-Isocyanate sind aus DE—OS 33 38 077 bekannt und können beispielsweise durch Phosgenierung von Dihydrooxaninen hergestellt werden.

Es ist möglich, das erfindungsgemäße Verfahren ohne Lösungsmittel durchzuführen.

Inerte Lösungsmittel für das erfindungsgemäße Verfahren sind bevorzugt polare Lösungsmittel, die sich unter den Reaktionsbedingungen nicht verärndern. Als Lösungsmittel werden insbesondere Chloroform, Tetrahydrofuran, Dioxan, Methylenchlorid, Toluol, Acetonitril und Freon bevorzugt. Als Lösungsmittel werden insbesondere bevorzugt Chloroform, Tetrahydrofuran, Freon und Acetonitril.

In einer besonderen Ausführungsform wird das erfindungsgemäße Verfahren unter Ausschluß von Wasser durchgeführt. Besonders bevorzugt wird eine maximals Menge von Wasser unter 0,1%.

Katalysatoren für das erfindungsgemäße Verfahren sind im allgemeinen Metallsalze höherer Fettsäuren. Bevorzugte Katalysatoren sind beispielsweise Dibutylzinnlaurat oder Zinn-(II)-octoat. Bevorzugt sind aber auch Verbindungen mit tertiären Aminogruppen, wie Pyridin, Methylpyridin, N,N'-Dimethylpiperazin, N,N-Dimethylbenzylamin und Titanverbindungen.

Im allgemeinen wird der Katalysator in einer Menge von 0,01 bis 2,5 Gew.%, bevorzugt von 0,1 bis 1,5 Gew.-%, bezogen auf die Gesamtmenge der Reaktanten eingesetzt.

In einer bevorzugten Ausführungsform kann das erfindungsgemäße Verfahren in Gegenwart eines Polymerisationsinhibitors durchgeführt werden. Ein Polymerisationsinhibitor kann beispielsweise 2,6-Di-tert.-butyl-4-methylphenol sein. Es ist aber möglich, Sauerstoff als Polymerisationsinhibitor zu verwenden. Hierbei leitet man Sauerstoff in das Reaktionsgemisch. Im allgemeinen ist der Luftsauerstoff ausreichend.

Im allgemeinen wird der Polymerisationsinhibitor in einer Menge von 0,01 bis 0,2 Gew.-%, bevorzugt von 0,05 bis 0,1 Gew.-% eingesetzt.

Die Tricyclodecane der Formel II werden im allgemeinen in einer Menge von 0,8 bis 1,4 mol, bevorzugt von 0,8 bis 1,2 mol, bezogen auf 2 mol der (Meth)-acrylsäureester-Isocyanate eingesetzt.

Das erfindungsgemäße Verfahren sind im allgemeinen im Temperaturbereich von 0 bis 100°C, vorzugsweise von 30 bis 70°C, durchgeführt. Das erfindungsgemäße Verfahren wird im allgemeinen bei

Normaldruck durchgeführt. Es ist aber auch möglich, das erfindungsgemäße Verfahren bei einem Unter- oder Überdruck (beispielsweise im Druckbereich von 0,1 bis 10 bar) durchzuführen.

Das erfindungsgemäße Verfahren kann beispielsweise wie folgt durchgeführt werden:

Die Reaktanten werden in dem Lösungsmittel gelöst und unter Rühren mit dem Katalysator und gegebenenfalls dem Polymerisationsinhibitor versetzt. Der zeitliche Veflauf de Umsetzung kann beispielsweise durch Messung der IR-Spektren verfolgt werden. Nach vollständiger Umsetzung der Isocyanatogruppen werden die Reaktionsprodukte durch Entfernen des Lösungsmittels isoliert. Eine vorherige Reinigung mit Hilfe von Adsorbentien, beispielsweise Aktivkohle, Bleicherde, Kieselgel oder Aluminiumoxid ist möglich.

Die erfindungsgemäßen (Meth)-acrylsäure-Derivate von Tricyclodecanen können als Monomere für Dentalmaterialien verwendet werden. So ist es möglich, sie als Monomere für polymere Zahnfüllmassen oder Beschichtungsmittel im Dentalbereich können die erfindungsgemäßen (Meth)-acrylsäure-Derivate von Tricyclodecanen können als Monomers für Dentalmaterialien verwendet werden. So ist es möglich, sie als Monomere für polymere Zahnfüllmassen oder Beschichtungsmittel (Zahnlacke) im Dentalbereich einzusetzen.

Für die Anwendung als Monomere für polymere Zahnfüllmassen oder Beschichtungsmittel im Dentalbereich können die erfindungsgemäßen (Meth)-acrylsäure-Derivate von Tricyclodecanen mit an sich bekannten Monomeren gemischt werden, um beispielsweise die Viskosität dem Verwendungszweck anzupassen. Viskositäten im Bereich vom 60 bis 10.000 mPas sind dabei bevorzugt. Dieses ist dadurch erreichbar, indem man den erfindungsgemäßen Monomeren gegebenenfalls ein Comonomer niedriger Visosität als Reaktivverdünner zumischt. Die erfindungsgemäßen Verbindungen werden in der Mischung mit Comonomeren mit einem Anteil von ca. 30 bis ca. 90 Gew.-%, eingesetzt, wobei ein Anteil von 50 bis 85 Gew.-% besonders bevorzugt ist.

Im Rahmen der vorliegenden Erfindung ist es ebenfalls bevorzugt, Mischungen verschiedener erfindungsgemäßer (Meth)-acrylsäure-Derivate von Tricyclodecanen einzusetzen.

Est ist auch möglich, Monomermischungen einzusetzen, die mehrere Comonomere als Reaktivverdünner enthalten.

Beispielsweise seien die folgenden Comomoneren genannt: Triethylenglykoldimethacrylat, Tetraethylenglykoldimethylacrylat, 1,12-Dodecandioldimethylacrylat, 1,6-Hexandioldimethacrylat, Diethylenglykoldimethacrylat, 2,2-Bis-[p-(2'-hydroxy-3'-methacrylolyloxypropoxy)-phenyl]-propan, 2,2-Bis-[p-(2'-methacrylolyloxypropoxy)-phenyl]-propan, Trimethylol-propan-tri-(meth)-acrylat, Bis-(meth)-acrylolyloxyethoxymethyl-tricyclo-[5.2.1.0$^{2,6}$]-decan (DE—OS 29 31 925 und DE—OS 29 31 926).

Insbesondere bevorzugt werden Comonomere, die bei 13 mbar einen Siedepunkt über 100°C besitzen.

Die erfindungsgemäßen (Meth)-acrylsäure-Derivate von Tricyclodecanen lassen sich gegebenenfalls in Mischungen mit den genannten Comonomeren mit an sich bekannte Methoden zu vernetzenden Polymerisaten aushärten (Am. Chem. Soc., Symp. Ser. *212*, 359—371 (1983)). Für die sogenannte Redoxpolymerisation ist ein System aus einer peroxidischen Verbindung und einem Reduktionsmittel, beispielsweise auf Basis tertiärer aromatischer Amine, geeignete.

Beispiele für Peroxide sind: Dibenzylperoxid, Dilaurylperoxid und Di-4-chlorobenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methyl-carbamoyloxypropyl)-3,5-dimethylanilin (DE—PS 27 59 239 genannt.

Die Konzentrationen des Peroxids bzw. des Amins werden vorteilhaft so gewählt, daß sie 0,1 bis 5 Gew.-% bevorzugt 0,5 bis 3 Gew.-%, bezogen auf die Monomermischung betragen. Die peroxid- bzw. aminhaltigen Monomermischungen werden bis zur Anwendung getrennt gelagert.

Die erfindungsgemäßen Monomeren können auch durch Bestrahlung mit UV-Licht ode sichtbarem Licht (beispielsweise im Wellenlägenbereich von 230 bis 650 nm) zur Polymerisation gebracht werden. Als Initiatoren für die photoinitierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwert von synergistisch wirkenden Photoaktivatoren, wie N,N-Dimethylaminoethylmethacrylat, Triethanolamin, 4-N,N-Dimethylaminobenzosulfonsäurediallylamid. Die Durchführung des Photopolymerisationsverfahrens ist beispielsweise in der DE—PS 31 35 115 beschrieben.

Neben den oben beschriebenen Initiatoren können den erfindungsgemäßen (Meth)-acrylsäure-Derivaten von Tricyclodecanen an sich für diesen Einsatzzweck bekannte Lichtschutzmittel und Stabilisatoren zugesetzt werden.

Lichtschutzmittel sind beispielsweise in (Grächter, Müller: Kunststoff-Additive, 2. Ausg., Carl Hauser Verlag, München, Wien) beschrieben. Beispielsweise seien die folgenden Lichtschutzmittel genannt: Cyasorbuva®, Tinuvin P®, Tinuvin 770®, Tinuvin 622®, Tinuvin 765®.

Stabilisatoren sind beispielsweise in (Ullmans Encyclopädie der technischen Chemie, Verlag Chemie Weinheim, 4. Aufl. Bd. 8) beschrieben. Beispielsweise seien die (folgenden Stabilisatoren genannt: 2,6-Di-tert.butylphenol, 2,6-Di-tert.butyl-4-methylphenol, 2,6-Di-octadecyl-4-methylphenol, 1,1'-Methylen-bis-(naphthol-2) u. a.

Das Lichtschutzmittel und der Stabilisator werden jeweils im allgemeinen in einer Menge von 0,01 bis 0,50 Gew.-%-Tln, bezogen auf 100 Gew.-%-Tle der Monomermischung, eingesetzt.

Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgmeinen Füllstoffe zu. Um einen hohen Füllgrad erreichen zu können, sind Monomermischungen, die eine Viskosität im Bereich von 60 bis 10.000 mPas besitzen, besonders vorteilhaft.

Den die erfindungsgemäßen Verbindungen enthaltenden Monomermischungen mischt man vorzugsweise anorganische Füllstoffe zu. Beispielsweise seien als anorganische Füllstoffe Bergkristall, Graphit, Kristoballit, Quarzglas, hochdisperse Kieselsäure, Aluminiumoxid und Glaskeramiken, beispielsweise Lantan und Zirkon enthaltende Glaskeramiken (DE—OS 23 47 501) genannt.

Die anorganischen Füllstoffe werden zur Verbesserung des Verbunds zur Polymermatrix beispielsweise eines Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings *11* 297—308 (1983)). Bevorzugt wird 3-Methacrylolyloxipropyltrimethyloxysilan eingesetzt.

Die Füllstaffe für die erfindungsgemäßen Zahnfüllmassen weisen im allgemeinen einen mittleren Teilchendurchmesser von 0,01 bis 100 µm, vorzugsweise von 0,05 bis 50 µm, besonders bevorzugt von 0,05 bis 5 µm. Es kann auch vorteilhaft sein, mehrere Füllstoffe nebeneinander einzusetzen, die einen voneinander verschiedenen Teilchendurchmesser besitzen.

Der Füllstoffanteil in der Zahnfüllmasse beträgt im allgemeinen 5 bis 85 Gew.-%, vorzugsweise 50 bis 80 Gew.-%.

Für die Herstellung de Zahnfüllmassen werden die Komponenten unter Verwendung handelsüblicher Knetmaschinen verarbeitet.

Der Anteil der erfindungsgemäßen (Meth)-acrylsäure-Derivate von Tricyclodecanen in den Füllmassen beträgt im allgemeinen 5 bis 85 Gew.-%, bevorzugt 15 bis 50 Gew.-%, bezogen auf die Füllmasse.

Dentalwerkstoffe auf Basis der erfindungsgemäßen (Meth)acrylsäure-Derivate von Tricyclodecanen haben überraschenderweise einen niedrigen Polymerisationsschrumpf und eine hohe mechanische Festigkeit.

Herstellungsbeiespiele:

1) Umsetzung von 3(4),8(9) Dihydroxymethyl-tricyclo-[5.2.1.$0^{2,6}$]-decan mit 2-Isocyanatoethylmethacrylat

In einem geeignet ausgerüsteten Reaktionsgefäß werden unter Rühren und Durchleiten von trockener Luft 0,3 Mol 3(4),8(9) Dihydroxymethyl-tricyclo-[5.2.1.$0^{2,6}$]-decan, 0,6 Mol 2-Isocyanatoethylmethacrylat, 0,2 g Dibutylzinndilaurat und 50 mg 2,6-Di-tert.-butyl-4-methyl-phenol (Ionol) in 3 h bei 70°C zur Reaktion gebracht. Man erhält 151 g einer farblosen, leicht viskosen Flüssigkeit.

$^1$H—NMR (ppm) in $CDCl_3$/TMS: 0,75—2,70 (14H); 1,95 (4H), 3,30—3,60 (4H); 3,70—3,95 (4H), 4,10—4,30 (4H); 50—530 (2H); 5,50—5,65 (2H); 6,08—6,15 (2H).

Viskosität (25°C): 885 Pas.

2) Umsetzung von 3(4),8(9) Dihydroxymethyl-tricyclo-[5.2.1.$0^{2,6}$]-decan mit 1-Isocyanato-2-methyl-but-3-yl-acrylat

0,25 Mol 3(4),8(9) Dihydroxymethyl-tricyclo-[5.2.1.$0^{2,6}$]-decan, 0,5 Mol 1-Isocyanato-2-methyl-but-2-yl-acrylat, 0,2 g Dibutylzinndilaurat, 50 mg Ionol werden wie in 1 beschrieben zur Reaktion gebracht.

$^1$H—NMR (ppm) in $CDCl_3$/TMS: 0,85—1,05 (6H), 1,15—1,35 (6H), 0,75—2,70 (14H), 2,70—3,55 (4H), 3,70—3,95 (4H), 4,70—5,30 (4H), 5,70—6,55 (6H).

Viskosität (25°C): 1120 Pas.

Anwendungsbeispiele

3) Messung des Polymerisationsschrumpfes:

Im reinen Monomer werden 2% Benzoylperoxid gelöst. 5 g dieser Lösung werden in ein zylindrisches Glasgefäß von 3 cm Durchmesser gefüllt und mit Stickstoff überschichtel. Die Lösung wird 1 h auf 80°C und 15 min. auf 130°C erhitzt, wobei die Monomere polymerisieren. Von den erhaltenen Probekörpern wird die Dichte bestimmt und durch Vergleich mit der Dichte der flüssigen Monomeren der Polymerisationsschrumpf bestimmt.

<u>Tabelle I</u>

| Vergleichsversuche Monomer | Polymerisationsschrumpf |
|---|---|
| 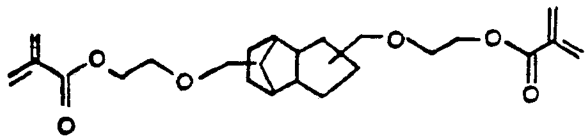 | 7,3 % |
| (DE-OS 29 31 926) | |
| 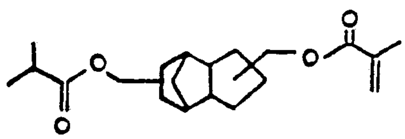 | 7,7 % |
| (DE-OS 28 16 823) | |
| **Erfindungsgemäße Monomere** | |
| 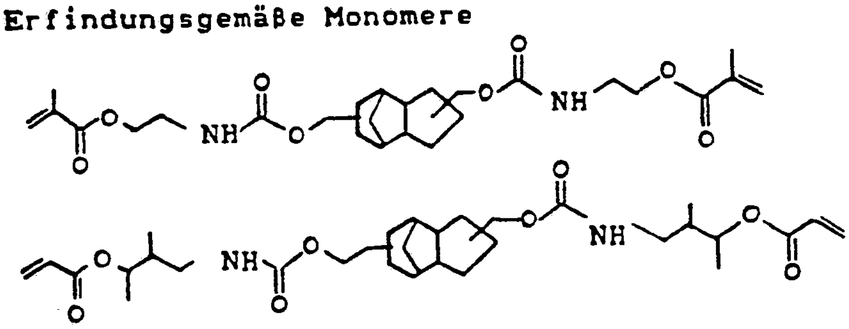 | 6,1 % |
| | 5,7 % |

4) Masse zum Füllen von Zahnholräumen

a) Redoxhärtendes System

*Peroxidpaste*

In einer Mischung aus 70 Teilen Monomer aus 1 und 30 Teilen Triethylenglycoldimethyacrylat werden 2% Benzoylperoxid gelöst.

10 g silanisiert Glaskeramik werden mit 4 g dieser Lösung zu einer Paste verarbeitet.

*Aminpaste*

In einer Mischung von 70 Teilen Monomer aus 1 und 30 Teilen Triethylenglycoldimethyacrylat werden 1,4% N-Methyl-N-β-(methylcarbamyloxy)propyl-3,5-dimethylanilin gelöst.

4 g dieser Lösung werden mit 10 g silanisierter Glaskeramik zu einer Paste verbeitet.

Werden gleiche Teile Aminpaste und Peroxidpastge miteinander gemischt, so härtet die Mischung in 2 Minuten aus. Die Pasten können mit Pigmenten eingefärbt werden und eignen sich Füllen von Zahnhohlräumen.

b) Lichthärtendes System

In einer Mischung von 70 Teilen Monomer aus 1 und 30 Teilen Triethylenglycoldimethyacrylat werden 0,5% N,N-Diallyl-p-dimethylaminobenzolsulfonsäureamid, 0,2% Campherchinon und 0,125% [4-N,N-Dimethylaminobenzolsulfonsäurediallylamid] gelöst. 10 g silanisiert Glaskeramik werden mit 4 g dieser Lösung zu einer Paste verarbeitet. Bestrahl man diese Masse mit einer handelsüblichen Dentallampe (Translux, Fa. Kulzer), so ist nach 40 sec. eine Schicht von 7,9 mm durchgehärtet.

5) Herstellung von Sealerlösungen

a) Redoxhärtendes System

*Katalysatorlösung*

In einer Mischung aus 10 Teilen Triethylenglycoldimethyl und 90 Teilen Monomer aus 2 werden 2% Benzoylperoxid gelöst.

*Aktivatorlösung*

In einer Mischung aus 10 Teilen Triethylenglycoldimethacrylat und 90 Teilen Monomer aus 2 werden 2% N-Methyl-N-β-(methylcarbamoyloxy)propyl-3,5-dimethylanilin gelöst.

Ein Mischung aus gleichen Teilen Katalysatorlösung und Aktivatorlösung härtet in 1 mikn 15 sec aus.

6) Lichthärtender Sealer

Im Monomeren aus 1 werden 0,5% N,N-Diallyl-p-dimethylaminobenzolsulfonsäureamid, 0,2% Campherchinon und 0,125% Benzildimethylketal gelöst. Beim Bestrahlen mit eine handelsüblichen Dentallampe (Translux, Fa. Kulzer) härtet die Flüssigkeit zu einem festen Film aus.

**Patentansprüche**

1. (Meth)-acrylsäure-Derivate von Tricyclodecanen der Formel

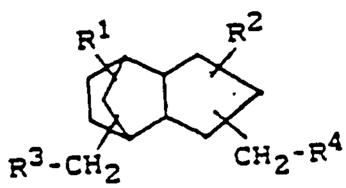

(I)

in der

$R^1$ und $R^2$ gleich oder verschieden sind und Wasserstoff, Niederalkyl Niederalkoxy, Halogen oder Trifluormethyl bedeuten, und

$R^3$ und $R^4$ gleich oder verschieden sind und für die Gruppe

$$-X-Y-O-\underset{\underset{O}{\|}}{C}-\underset{\underset{R^5}{|}}{C}=CH_2$$

stehen, wobei

X ein zweibindiges Brückenglied aus der Gruppe

$$-O-\underset{\underset{O}{\|}}{C}-NH- \quad \text{oder} \quad -\underset{\underset{R^6}{|}}{N}-\overset{\overset{O}{\|}}{C}-NH-$$

und

Y ein zweibindiges Brückenglied aus der Gruppe

$$-CH_2-CH_2- \quad \text{oder} \quad -\underset{\underset{CH_3}{|}}{CH}-\overset{\overset{CH_3}{|}}{CH}-CH_2- \quad \text{oder}$$

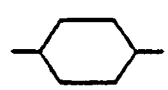

bedeutet,

und wobei

$R^5$ für Wasserstoff oder Methyl und

$R^6$ für Wasserstoff, Niederalkyl oder Phenyl stehen.

2. (Meth)-acrylsäure-Derivate von Tricyclodecanen nach Anspruch 1, wobei

$R^1$ und $R^2$ Wasserstoff bedeuten,

$R^3$ und $R^4$ gleich oder verschieden sind und für die Gruppe

$$-O-\underset{\underset{O}{\|}}{C}-NH-Y-O-\overset{\overset{O}{\|}}{C}-\underset{\underset{R^5}{|}}{C}=CH_2$$

stehen, wobei

Y ein zweibindiges Brückenglied aus der Gruppe

$$-CH_2-CH_2- \quad \text{oder} \quad -\underset{\underset{CH_3}{|}}{CH}-\overset{\overset{CH_3}{|}}{CH}-CH_2- \quad \text{oder}$$

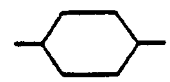

bedeutet,

und wobei

$R^5$ für Wasserstoff oder Methyl steht.

3. Verfahren zur Herstellung von (Meth)-acrylsäure-Derivate von Tricyclodecanen, dadurch gekennzeichnet, daß Tricyclodecane der Formel

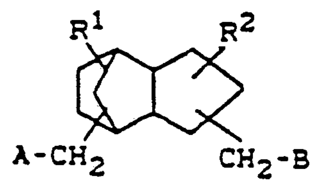

(II)

in der

$R^1$ und $R^2$ gleich oder verschieden sind und Wasserstoff, Niederalkyl Niederalkoxy, Halogen oder Trifluormethyl bedeuten, und

A und B gleich oder verschieden sind und Hydroxy oder den Rest —NHR$^6$ bedeuten, wobei $R^6$ für Wasserstoff, Niederalkyl oder Phenyl steht,

mit (Meth)-acrylsäureester-Isocyanaten der Formel

$$CH_2 = \underset{\underset{R^5}{|}}{C} - \underset{\underset{O}{||}}{C} - Y - NCO \qquad (III),$$

in der

$R^5$ für Wasserstoff oder Methyl und

Y ein zweibindiges Brückenglied aus der Gruppe

$$-CH_2-CH_2- \quad \text{oder} \quad -\underset{\underset{CH_3}{|}}{CH}-\overset{\overset{CH_3}{|}}{CH}-CH_2- \quad \text{oder}$$

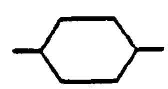

bedeutet,

gegebenenfalls in einem inerten Lösungsmittel in Gegenwart eines Katalysators im Temperaturbereich von 20 bis 100°C umsetzt.

4. Polymerisat aus (Meth)-acrylsäure-Derivaten von Tricyclodecanen der Formel

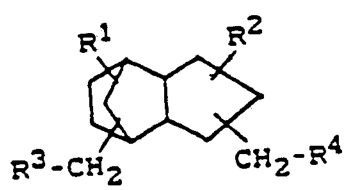

(I)

in der

$R^1$ und $R^2$ gleich oder verschieden sind und Wasserstoff, Niederalkyl Niederalkoxy, Halogen oder Trifluormethyl bedeuten, und

$R^3$ und $R^4$ gleich oder verschieden sind und für die Gruppe

$$- X - Y - O - \underset{\underset{O}{||}}{C} - \underset{\underset{R^5}{|}}{C} = CH_2$$

stehen, wobei

X ein zweibindiges Brückenglied aus der Gruppe

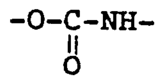 oder 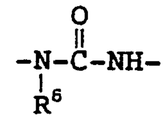

und

Y ein zweibindiges Brückenglied aus der Gruppe

$-CH_2-CH_2-$ oder 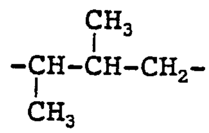 oder 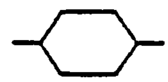

bedeutet,

und wobei

$R^5$ für Wasserstoff oder Methyl und

$R^6$ für Wasserstoff, Niederalkyl oder Phenyl stehen.

5. Verwendung von (Meth)-acrylsäure-Derivaten von Tricyclodecanen nach Anspruch 1 in Dentalwerkstoffen.

6. Verwendung nach Anspruch 5, dadurch gekennzeichnet, daß die (Meth)-acrylsäure-Derivate von Tricyclodecanen in Zahnfüllmassen eingesetzt werden.

7. Verwendung nach Anspruch 6, dadurch gekennzeichnet, daß die (Meth)-acrylsäure-Derivate von Tricyclodecanen in Beschichtungsmittel für Zähne eingesetzt werden können.

8. Zahnfüllmassen, dadurch gekennzeichnet, daß sie (Meth)-acrylsäure-Derivate von Tricyclodecanen der Formel

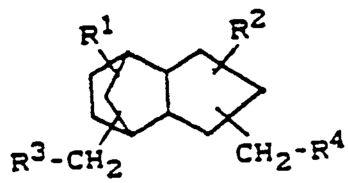 (I)

in der

$R^1$ und $R^2$ gleich oder verschieden sind und Wasserstoff, Niederalkyl, Niederalkoxy, Halogen oder Trifluormethyl bedeuten, und

$R^3$ und $R^4$ gleich oder verschieden sind und für die Gruppe

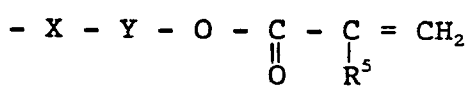

stehen, wobei

X ein zweibindiges Brückenglied aus der Gruppe

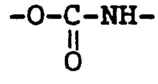 oder 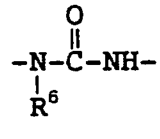

und

Y ein zweibindiges Brückenglied aus der Gruppe

$-CH_2-CH_2-$ oder 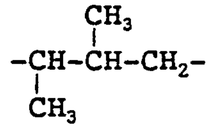 oder 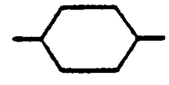

bedeutet,

und wobei

$R^5$ für Wasserstoff oder Methyl und

$R^6$ für Wasserstoff, Niederalkyl oder Phenyl stehen, enthalten.

9. Zahnfüllmassen nach Anspruch 8, dadurch gekennzeichnet, daß sie neben (Meth)-acrylsäure-Derivaten von Tricyclodecanen ein weiteres Comonomer enthalten.

**Revendications**

1. Dérivés (méth)-acryliques de tricyclodécanes, répondant à la formule

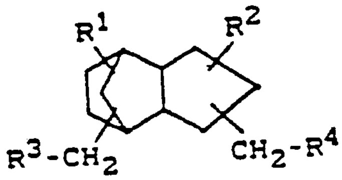

(I)

dans laquelle

$R^1$ et $R^2$ sont identiques ou différents et représentent un atome d'hydrogène, un groupe alkyle inférieur, un groupe alcoxy inférieur, un atome d'halogène ou un groupe trifluorométhyle, et

$R^3$ et $R^4$ sont identiques ou différents et représentent le groupe

$$-X-Y-O-\underset{\underset{O}{\parallel}}{C}-\underset{\underset{R^5}{|}}{C}=CH_2$$

où

X représente un pont à deux liaisons choisi dans le groupe

$$-O-\underset{\underset{O}{\parallel}}{C}-NH- \quad \text{ou} \quad -\underset{\underset{R^6}{|}}{N}-\overset{\overset{O}{\parallel}}{C}-NH-$$

Y représente un pont à deux liaisons choisi dans le groupe

$$-CH_2-CH_2- \quad \text{ou} \quad -\underset{\underset{CH_3}{|}}{CH}-\overset{\overset{CH_3}{|}}{CH}-CH_2- \quad \text{ou}$$

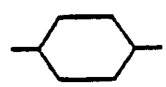

et où

$R^5$ représente un atome d'hydrogène ou un groupe méthyle et

$R^6$ représente un atome d'hydrogène, un groupe alkyle inférieur ou un groupe phényle.

2. Dérivés (méth)-acryliques de tricyclodécanes, selon la revendication 1, dans lesquels

$R^1$ et $R^2$ représentent un atome d'hydrogène,

$R^3$ et $R^4$ sont identiques ou différents et représentent le groupe

$$-O-\underset{\underset{O}{\parallel}}{C}-NH-Y-O-\overset{\overset{O}{\parallel}}{C}-\underset{\underset{R^5}{|}}{C}=CH_2$$

où

Y représente un pont à deux liaisons choisi dans le groupe

$$-CH_2-CH_2- \quad \text{ou} \quad -\underset{\underset{CH_3}{|}}{CH}-\overset{\overset{CH_3}{|}}{CH}-CH_2- \quad \text{ou}$$

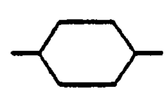

et où

$R^5$ représente un atome d'hydrogène ou un groupe méthyle.

3. Procédé de préparation de dérivés (méth)-acrylique, de tricyclodécanes caractérisé en ce que l'on fait réagir des tricyclodécanes répondant à la formule

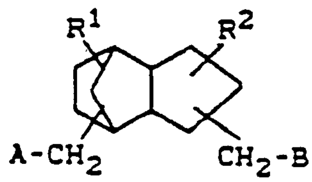

(II)

dans laquelle

$R^1$ et $R^2$ sont identiques ou différents et représentent un atome d'hydrogène, un groupe alkyle inférieur, un groupe alcoxy inférieur, un atome d'halogène ou un groupe trifluorométhyle, et

A et B sont identiques ou différents et représentent un groupe hydroxyle ou le radical —$NHR^6$, ou $R^6$ représente un atome d'hydrogène, un groupe alkyle inférieure ou un groupe phényle, avec des isocyanates d'esters (méth)acryliques de formule

$$CH_2 = \underset{|\;R^5}{C} - \underset{\|\;O}{C} - Y - NCO \qquad (III),$$

dans laquelle

$R^5$ représente un atome d'hydrogène ou un groupe méthyle et

Y représente un pont à deux liaisons choisi dans le groupe

$$-CH_2-CH_2- \quad \text{ou} \quad -\underset{|\;CH_3}{CH}-\overset{CH_3\;|}{CH}-CH_2- \quad \text{ou}$$

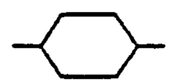

éventuellement dans un solvant inerte, en présence d'un catalyseur, dans un intervalle de température allant de 20 à 100°C.

4. Polymérisat constitué de dérivés (méth)-acryliques de tricyclodécanes, répondant à la formule

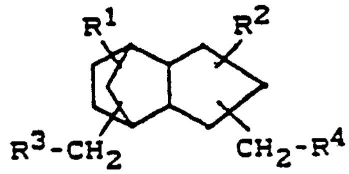

(I)

dans laquelle

$R^1$ et $R^2$ sont identiques ou différents et représentent un atome d'hydrogène, un groupe alkyle inférieur, un groupe alcoxy inférieur, un atome d'halogène ou un groupe trifluorométhyle, et

$R^3$ et $R^4$ sont identiques ou différents et représentent le groupe

$$- X - Y - O - \underset{\|\;O}{C} - \underset{|\;R^5}{C} = CH_2$$

où

X représente un pont à deux liaisons choisi dans le groupe

$$-O-\underset{\|\;O}{C}-NH- \quad \text{ou} \quad -\underset{|\;R^6}{N}-\overset{O\;\|}{C}-NH-$$

et

Y représente un pont à deux liaisons choisi dans le groupe

$-CH_2-CH_2-$ ou 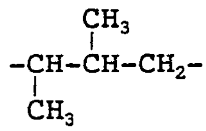 ou 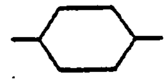

et où

$R^5$ représente un atome d'hydrogène ou un groupe méthyle et

$R^6$ représente un atome d'hydrogène, un groupe alkyle inférieur ou un groupe phényle.

5. Utilisation de dérivés (méth)-acryliques de tricyclodécanes selon la revendication 1, dans des matériaux dentaires.

6. Utilisation selon la revendication 5, caractérisée en ce qu'on incorpore les dérivés (méth)-acryliques de tricyclodécanes dans des matières d'obturation dentaire.

7. Utilisation selon la revendication 6, caractérisée en ce qu'on peut incorporer les dérivés (méth)-acryliques de tricyclodécanes dans des agents d'enduction pour les dents.

8. Matières d'obturation dentaire, caractérisées en ce qu'elles contiennent les dérivés (méth)-acryliques de tricyclodécanes, répondant à la formule

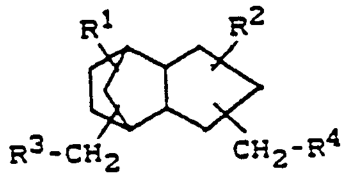

(I)

dans laquelle

$R^1$ et $R^2$ sont identiques ou différents et représentent un atome d'hydrogène, un groupe alkyle inférieur, un groupe alcoxy inférieur, un atome d'halogène ou un groupe trifluorométhyle, et

$R^3$ et $R^4$ sont identiques ou différents et représentent le groupe

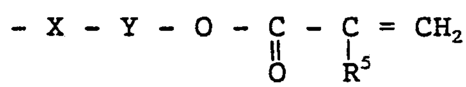

où

X représente un pont à deux liaisons choisi dans le groupe

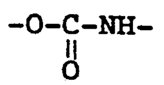 ou 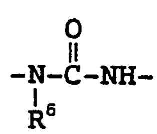

et

Y représente un pont à deux liaisons choisi dans le groupe

$-CH_2-CH_2-$ ou 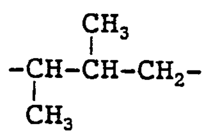 ou 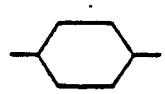

et où

$R^5$ représente un atome d'hydrogène ou un groupe méthyle et

$R^6$ représente un atome d'hydrogène, un groupe alkyle inférieur ou un groupe phényle.

9. Matières d'obturation dentaire selon la revendication 8, caractérisées en ce qu'elles contiennent un autre comonomère, en plus des dérivés (méth)-acryliques de tricyclodécanes.

**Claims**

1. (Meth)-acrylic acid derivatives of tricyclodecanes of the formula

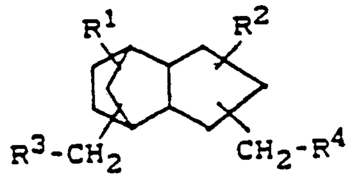

(I)

in which

$R^1$ and $R^2$ are identical or different and denote hydrogen, lower alkyl, lower alkoxy, halogen or trifluoromethyl and

$R^3$ and $R^4$ are identical or different and represent the group

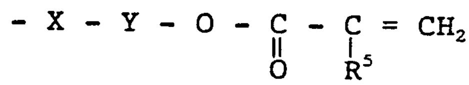

wherein

X denotes a divalent bridge member from the group comprising

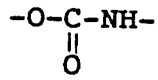 and 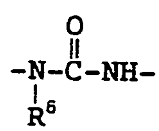

and Y denotes a divalent bridge member from the group comprising

$-CH_2-CH_2-$ and 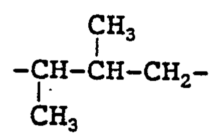 and 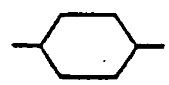

and wherein

$R^5$ represents hydrogen or methyl and

$R^6$ represents hydrogen, lower alkyl or phenyl.

2. (Meth)-acrylic acid derivatives of tricyclodecanes according to Claim 1, wherein

$R^1$ and $R^2$ denote hydrogen,

$R^3$ and $R^4$ are identical or different and represent the group

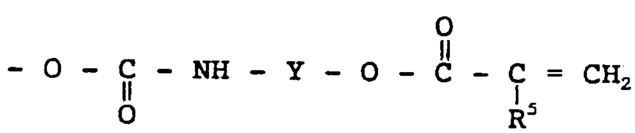

wherein

Y denotes a divalent bridge member from the group comprising

$-CH_2-CH_2-$ and 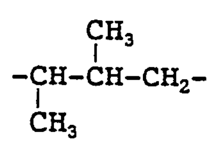 and 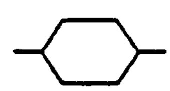

and wherein

$R^5$ represents hydrogen or methyl.

3. Process for the preparation of (meth)-acrylic acid derivatives of tricyclodecanes, characterized in that tricyclodecanes of the formula

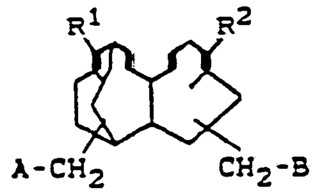

(II)

in which

$R^1$ and $R^2$ are identical or different and denote hydrogen, lower alkyl, lower alkoxy, halogen or trifluoromethyl and

A and B are identical or different and denote hydroxyl or the radical —$NHR^6$, wherein

$R^6$ represents hydrogen, lower alkyl or phenyl, are reacted with (meth)-acrylic acid ester-isocyanates of the formula

$$CH_2 = \underset{R^5}{\underset{|}{C}} - \underset{O}{\underset{\|}{C}} - Y - NCO \qquad (III),$$

in which

$R^5$ represents hydrogen or methyl and

Y denotes a divalent bridge member from the group comprising

$-CH_2-CH_2-$ and $-\underset{CH_3}{\underset{|}{CH}}-\overset{CH_3}{\overset{|}{CH}}-CH_2-$ and 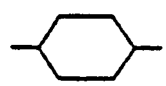

if appropriate in an inert solvent in the presence of a catalyst in the temperature range from 20 to 100°C.

4. Polymer of (meth)-acrylic acid derivatives of tricyclodecanes of the formula

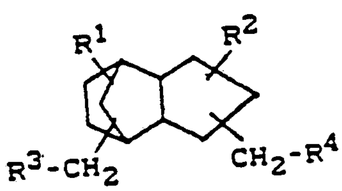

(I)

in which

$R^1$ and $R^2$ are identical or different and denote hydrogen, lower alkyl, lower alkoxy, halogen or trifluoromethyl and

$R^3$ and $R^4$ are identical or different and represent the group

$$- X - Y - O - \underset{O}{\underset{\|}{C}} - \underset{R^5}{\underset{|}{C}} = CH_2$$

wherein

X denotes a divalent bridge member from the group comprising

$-O-\underset{O}{\underset{\|}{C}}-NH-$ and $-\underset{R^6}{\underset{|}{N}}-\overset{O}{\overset{\|}{C}}-NH-$

and Y denotes a divalent bridge member from the group comprising

$-CH_2-CH_2-$ and 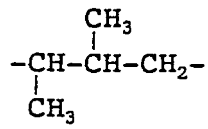 and 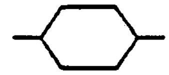

and wherein
$R^5$ represents hydrogen or methyl and
$R^6$ represents hydrogen, lower alkyl or phenyl.

5. Use of (meth)-acrylic acid derivatives of tricyclodecanes according to Claim 1 in dental materials.

6. Use according to Claim 5, characterized in that the (meth)-acrylic acid derivatives of tricyclodecanes are employed in dental filling compositions.

7. Use according to Claim 6, characterized in that the (meth)-acrylic acid derivatives of tricyclodecanes can be employed in coating agents for teeth.

8. Dental filling compositions, characterized in that they contain (meth)-acrylic acid derivatives of tricyclodecanes of the formula

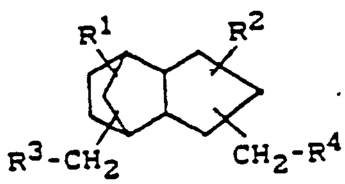 (I)

in which
$R^1$ and $R^2$ are identical or different and denote hydrogen, lower alkyl, lower alkoxy, halogen or trifluoromethyl and
$R^3$ and $R^4$ are identical or different and represent the group

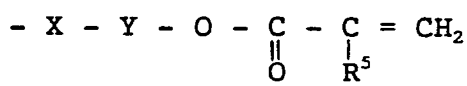

wherein
X denotes a divalent bridge member from the group comprising

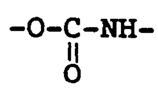 and 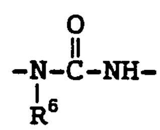

and Y denotes a divalent bridge member from the group comprising

$-CH_2-CH_2-$ and 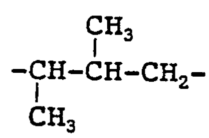 and 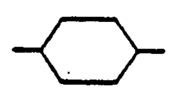

and wherein
$R^5$ represents hydrogen or methyl and
$R^6$ represents hydrogen, lower alkyl or phenyl.

9. Dental filling compositions according to Claim 8, characterized in that, in addition to (meth)-acrylic acid derivatives of tricyclodecanes, they contain another comonomer.